# EUROPEAN PATENT APPLICATION

(11) **EP 1 475 436 A1**
(43) Date of publication of application: **10.11.2004**
(21) Application number: 03703156.4
(22) Date of filing: 04.02.2003
(51) Int. Cl.: C12N 15/09, C12N 5/16, C07K 14/47, C07K 16/18, A01K 67/00

(54) **SWINE RAG-1 GENE AND UTILIZATION THEREOF**

(30) Priority: 04.02.2002 JP 2002027450
(71) Applicant: National Institute of Agrobiological Sciences, Tsukuba-shi, Ibaraki 305-8602 (JP)
(72) Inventor: YASUE, Hiroshi, Tsukuba-shi, Ibaraki 305-0032 (JP); WATANABE, Satoshi, Tsukuba-shi, Ibaraki 305-0044 (JP); HONMA, Daisuke, Ushiku-shi, Ibaraki 300-1232 (JP); TAKAGAKI, Yohtaroh, Kamakura-shi, Kanagawa 248-0014 (JP); HATSUSE, Hiromi, Yokohama-shi, Kanagawa 241-0801 (JP)
(74) Representative: Winckels, Johannes Hubertus F.
(86) International application number: PCT/JP2003/001114
(87) International publication number: WO 2003/066855

(57) **Abstract**

BAC clones comprising the porcine RAG-1 gene were obtained by screening a porcine genomic DNA library. Three clones were obtained by subcloning the region comprising the RAG-1 gene from the BAC clones. Genetic maps were obtained by determining the nucleotide sequence of these clones using sequencing, and by mapping using restriction enzyme digests. Based on this information, targeting vectors that can knockout the RAG-1 gene were constructed. Using these vectors it is possible to construct swine that do not possess acquired immunity by suppressing endogenous porcine RAG-1 gene function.

SEQUENCE LISTING

<110> National Institute of Agrobiological Sciences

<120> PORCINE RAG-1 GENES AND USES THEREOF

<130> MOA-A0111P

<140>
<141>

<150> JP 2002-27450
<151> 2002-02-04

<160> 3

<170> PatentIn Ver. 2.1

<210> 1
<211> 9098
<212> DNA
<213> Sus.scrofa

<220>
<221> exon
<222> (827)..(924)

<220>

<221> intron

<222> (925)..(5364)

<220>

<221> exon

<222> (5365)..(9098)

<400> 1

gagctcctgt tgtggctcag taggttaaga acccagcaca gggtctgtga ggattcgggt 60

tcaatccttg gtctcgatca gtgggttaag gatccgtcat tgccgtgagc tgtggtgtag 120

gttgcggatg caagtggatc cggtgtggct gtggtgcagg ctggcagatg caactccaat 180

tccaccccta acccaggaac ttccatatgc cgcaggtgca gtcctgaaaa gaaaagaaag 240

gaatggaaaa gcaaggaaag gaaaggaaaa gaaaagaact ccagtaatgt aaacatggtg 300

ttgttgcaag gagcacagaa ttccctgact gagaactgac cagggaggag aattctgcgg 360

gtggtttggt taccatttca aaattataat aaaaagtgct cagatagtta gcatttgatg 420

agtatctgct aaaggtaaac actgtcccaa gtcttctcct ataacaagtc tcactataac 480

ctcattgact ctcagaaatg actcttcaga tttccagctt gcaggtgggg aaactaaggc 540

acagagctgc gggagcttag cacaagcccg agcttatgtt gacaagatca tctccaaccc 600

aacagccttg gggagcgaac tggcagcttt ggcagtgctc cagcacacac ccatgtgtgg 660

gtcttcagat cccccagtta agcttcctga agagccaggt gccagctgga gctgggggct 720

cctggctggt gattggctgc catcactggt ggttagccct ctggggtggt gggggggacc 780

gggaaggaca ggggaggctg ataaataaag cagccatctg ctctgaggga tgggagagca 840

cattttgcct tctctttggt agcgagtaat atcaaccgaa gtgcagacat cctaacactt 900

tgcccaggca gcctgctgag caaggtaaca ctcatccttt tcatgccttg agtcacaata 960

tttatgacat ttttatgttt ctcattagaa gtacttgggc tttgttcctt gcggggaatg 1020

agaggaggga gcaagggacg ctggcgtaca tttgactttt cttgctaatg tacgaaaata 1080

ggaaaggaac cagaaaccaa gttgaggaag acactgtacg ttcaaagaga agacggaaaa 1140

tatctgaatt actgtaaata aatgctgcag gcagaggaag tgtttaagga gtgcttcttg 1200

agtggatgtg taaagcaaat cagcagaagg agaggtgtga gactcttgaa aaaaatattg 1260

tgctggtcca ggtaataaga gtaatcagga gcaaacaaat tccagggcgg gaggaaatgt 1320

acttgcccag gtatttgcca gggtagtgcc aggaatttcc tgaattatat aggaccagga 1380

gaccagtatt tctcagtgtg tgacctgtgg gccactgaaa tacagcagga tcattgaaat 1440

gcttgttaaa aaatatgatc tctggagttc ccgtcgtggc gcagtggtta acgaatccga 1500

ctaggaacca tgaggttgcg ggttcggtcc ctgcccttgc tcagtgggtt aacgatccgg 1560

cgttgccgtg agctgtggtg taggttgcag acgtggctcg gatcccgcgt tgctgtggct 1620

ctggcgtagg ccggtggcta cagctccgat tcgaccccta gcctgggaac ctccatatgc 1680

cgcgggagcg gcccaagaaa tagcaacaac aacaacaaca acaaaagaca aaagacaaaa 1740

aaaaaaaaaa aaaaatatga tctctggact ccaccccaga taaattgaat cagattctct 1800

gggacctgac gatctgcctt atttttttaa acccaagttc cctagtgatt ctgatacaga 1860

gcaattttga gaatgccacg gtagagccct acacagaggt tatgttcttg aacagggttc 1920

ctgaccaggg gctgaagtgc ttcaagcaag aagttgcctg gaggaagcgt aagaatcttc 1980

aggtcatctt gtagatgcct gagacttata aaaggctttt gatactatca ttttttgatg 2040

atggtgtttg gcaaagagta gacaataata tttgcggagt gaatgaatga gggattaagt 2100

gggacaggtg tgacaataaa ggatcacacc tgggcacact gcagtcactc aaactttgtg 2160

tgtggaccaa gtggaatatg ttaagaggag agatgactga ccaaggccca gctcggaggt 2220

gtgtgtaagg tgtgtgtgtc gggggagctt ggcagggagg tggagggggc gctgctgtaa 2280

ctctgccccc tgaggttact gatgattcac taacacaaat aacagcttct gaccctgcct 2340

gacttgtcaa aatgaatccg tgcccttaga gagaaggccc ctggttgctg tgatggcaag 2400

tgatggcaat actcagaggg cagagcccga tgggaacagc tgagccagca gcaaccaccc 2460

tgccttgtag agtctggtcc actgctgaaa ccttctgtta gaagagagga aatcaagcat 2520

tcacaaaatt cctgagtgtc aaaaaaaaaa aaaaaaaaaa aaaaaagaaa gaaagaacga 2580
aaatgactac aagataaccc ataatcctaa ctttatgtgc tgtcaacagg tccctctttt 2640
agtccccttt ctgcgtgttt accagaagag aaaggtaagc taagaatggt agtcagattt 2700
gcagcttagc caattaaaaa atttttttat tatagttgat ttatattgtt ctgccaattt 2760
ctgccgttaa cttagccaaa tcttaaattt ttctgggggg aaactataga aaagtgacct 2820
tggtacatag aaacctaggc tttaaaacat ttttttcccc taaggtgaat agatcacaaa 2880
aattaaatat aaatataaag cagttgagta gaaaagcaat ctaataaata aatttgattt 2940
ggatgtcaac gctagtaact gcatggtgat ggtactcttt ccatggtagt atttacacaa 3000
atatagttta ggcagatttt gatgcaacat ttcaggccac tgggagtcag gagaggattg 3060
ggtagggaga ggaggcagtt ctagggatcc ttccagtatg ccttgaggtt gactgactca 3120
ttgtgaaagt tcaatatttc atttgattag ctctacagat gaccatgcaa ttattcataa 3180
gaactcaaaa tatcgtctcg gaattccaga gaactagtgc attagaaaag gcagggaaag 3240
agttccctgg tagctcaatg ggttaaggac ccagcattgt ctctgttgtg gctcaggtca 3300
ctgctgtgat gtcggttcaa tccttggccc agacacaggg aaaggaaggg ctaaagaaac 3360
aaaacacatg gaaataagtt caaaccagtg ggacagaagt gacatccttg ccgctgatcc 3420
tgaatgtagg aacataactg aacaataagc aactttgagg cgttggtcgg actgcagttg 3480
aaatactttt gaggttaatg aggcctttga aatggtttgg aactgtttag ttctcacaca 3540
gtcctgcata tagaacagtg taacaggatt tccttttcaa ccaagacttg gaaattggct 3600
tttcgtcttt atgaaaaaca tgaatctctg aagcatcatg catgcaagat ggaacaaact 3660
tctgtggttg aaagggatga cctaacagga agctttgtta gcagcagtca ggatcaccct 3720
tgtggctttc ctttcccctc ccccaaatcc cacccctggg caaagtgtgt tcatcagagg 3780
attagtctcc ataccacagg acaataacaa tatcatcaac ttggttagtt agttaattga 3840
aggggaagca ttgtttgata taattggcgg gtcaagagca ctaacccagg aaaatatgga 3900
aaatttagat tgtagtttga aaatacaatt gctgtttttc cagtcatgct gatactaagg 3960
aattttgaga atattgtgct aattgggtct agagacactc aaatggcatt tttaaacttt 4020
tttctacagc tctgttgaag cctacaccac aacccaggtt gctagggttt cctggaatat 4080

ataatggcca atgttttttt tgaagaagaa aattattact cccacctctt cttattacaa 4140
acgatagagc taatgaccag tgcccagtaa tgacttttgg gatggcctgt gtgaattcga 4200
cctgaaacta cttgaatttt tcatcttaaa agtcctttgg agtctctgta catgcacact 4260
gtggtttgtt gcaggtttag agagttctgt gggttttgtt cctaatggag acacagcctt 4320
ataatttaca gcagcgttca ctaattaaaa ttgtaagcat aattactatc catgatactt 4380
gttattagtg tgcattcata aagctcaaaa ttcacttcat cctttcaagt agcgagtaat 4440
tagtttcttt ggatttgtag ctttatcatc cctttatgtc ccgtttcaga gaaataaaca 4500
accaacctcc tggcagactg ctttaaaaag ctggaaatac attgtccagc tagtgcaatg 4560
agactgatac agcgcggaaa atatgacttt tctttggttt tagtaaccag tttttccttg 4620
catttattca gcagataact attgagcatc taatgtgtgc tagaaagctt gggggagaca 4680
gagataaatc actcaaagat tccgtcctaa aaaacgtcca ggttttggtg gaaaggaaat 4740
aatatcgtac atagataata ttgtacatca actatatctc aataaaaaag aataattttt 4800
ataaaagaaa tgattgtaca tatatacgta aaatagacca tggtgatgat gcgtgaacgc 4860
agaaacatgt aaatggacaa tgccactgaa agaacaggaa atgactctgt tttctgtata 4920
tgatcagccc taacatagta tattaacata ggaagccaaa caattaactt tctgaatgct 4980
ttaataaatg aataggcgtt cccattgtgg cgcagtggtt aacgaatcca actaggaacc 5040
atgaggttgt gggttcgatc cctgcctttg ctcagtgggt taaggagccg gtattgccat 5100
gatctgtggt gtaggtcgca gatgcggctc aggtcccaag cagctgtggc tctggcgtag 5160
gccagcggct gcagctcccc tagcctggga acctccgtgt gctggttctg aaaaaaggca 5220
aaaagacaaa aaaataaaat aaataataaa ataaaataaa aaataaatga ataattgaat 5280
gtactaattg gtattccttc caagttgcat cagtgggtta tttacatttt ttttcctaac 5340
ctgacttgtc cttattgctc ccaggtacct cagccagcat ggctgtctct ttgccaccca 5400
ctctgggact cagttccgcc ccagatgaaa tccagcaccc ccacattaaa ttttcagaat 5460
ggaaatttaa gctattcagg gtgagatcct ttgaaaaggc acctgaaaag gctcaaacgg 5520
aaaagcagga ttcctccgag gggaaaccct cgctggagca atctccagca gtcctggaca 5580
agcctggtgg tcagaagtca gccctgcctc aaccagcatt caagccccat ccaaagtttt 5640

taaaggaatc ccacgaagat gggaaagcaa gagacaaagc catccaccaa gccaacctga 5700
gacgtctctg ccgcatctgt gggaattctt tcaacaccac tgggcacaag agaaggtatc 5760
cagtccacgg gcctgtggat ggtaaaaccc aagtcctttt acggaagaag gaaaagaggg 5820
ccacgtcctg gccagacctc attgccaaag ttttccggat cgatgtgaag gcagatgttg 5880
actcgatcca ccccactgag ttctgccata actgctggag cttcatgcac aggaagttta 5940
gcagcacccc atgtgaggtt tactccccaa ggaatgcagc catggagtgg cacccccaca 6000
ccctaaactg tgacatctgc cacattgcac gtcggggact caagaggaag agtcagcagc 6060
caaacatgca gctcagcaaa aaactcaaaa ctgtgattga ccgagcgaga caagcccgtc 6120
agcgcaagag gagagctcag gccaggatca gcagcaagga actgatgaag aagatcgcca 6180
actgcggtca gatacatctt agccccaagc tcctggcagt ggacttcccg gcgcactttg 6240
tgaaatctat ctcctgccag atttgtgaac acatcctggc cgacccggtg gagaccagct 6300
gcaagcatgt gttttgcagg atctgcattc tcaggtgcct caaagtcatg ggcagcagtt 6360
gtccctcttg ccactatccc tgttttccta ctgacctgga gagtccagtg aagtcttttc 6420
tgagcatctt gaataccctg atggtgaaat gcccagcaaa ggagtgcaac gaggagatca 6480
gcttggaaaa atataatcac catatctcaa gccacaagga gtcgaaggag acatttgtgc 6540
atattaataa agggggccgg ccccgccagc atctcctgtc cctgacgcgg agggctcaga 6600
aacaccgtct gagggagctc aagctgcaag tcaaggcctt cgccgacaaa gaagaaggtg 6660
gcgatgtgaa gtcagtgtgc ctgaccttgt tcctgctagt gctgagggcg aggaatgagc 6720
acagacaagc tgacgagctg gaggccatca tgcgaggcca gggctccggc ctgcagcctg 6780
ctgtttgctt ggccatccgc gtcaacacct tcctcagctg cagccagtac cacaagatgt 6840
acaggactgt gaaggccatc acgggcaggc agattttcca gcctttgcat gcccttcgga 6900
atgcggagaa ggtccttctg cccggctacc accccttcga gtggcagcca cctctgaaga 6960
atgtgtcttc cagcacagac gtgggcatta ttgatgggct gtctggactc tcctcctctg 7020
tggacgatta cccagtggac accattgcca agcgcttccg ctatgactcg gctctggtgt 7080
ccgctctcat ggacatggaa gaagacatcc tggagggtat gagagcccaa gaccttgacg 7140
actacctgaa tggccccttc actgtggtgg tgaaggagtc ttgtgatggg atgggagacg 7200

tgagtgagaa gcacggcagt gggccggtcg tgccggaaaa ggccgttcgg ttttccttca 7260
cagtcatgaa aatcaccatc gcacacgggt cacagaacgt gaaggtgttt gaggaagcca 7320
agcctaactc tgaactatgc tgcaagccct tgtgcctcat gctggccgac gaatccgacc 7380
atgagaccct gacggccatc ctgagccctc tcattgccga gagggaggcc atgaagagca 7440
gccagctaat gctggagatg ggaggcatcc tccggacttt caagttcatc ttcaggggca 7500
ccggatatga tgagaaactg gtccgggaag tggaaggcct tgaggcttct ggctctgtct 7560
acatctgtac cctctgtgat gccacccgcc tggaagcctc tcaaaatctg gtcttccact 7620
ccataaccag aagccacgcg gagaatttgg agcgctatga ggtctggcgt tccaacccat 7680
accatgagac ggtggatgaa cttcgggacc gggtgaaagg ggtctcggcc aaacccttca 7740
ttgagacggt gccttccata gatgccctcc actgtgacat tggcaatgca gccgagtttt 7800
acaagatttt ccagctcgag ataggggagg cgtataagaa cccccatgcc tccaaggagg 7860
aaaggaagag atggcaggcg accttggaca agcacctccg caagaagatg aatctgaagc 7920
ccatcatgag aatgaatggc aactttgcca ggaagctcat gaccaaagag actgtggaag 7980
cagtctgtga gttaattccc tccgaggaga ggcatgaagc tctgagggaa ctgatggacc 8040
tttacctgaa gatgaagccc gtctggcgat catcatgccc tgctaaagag tgcccggaat 8100
ccctctgcca gtatagtttc aattcacagc gttttgctga gctcctctcc accaagttca 8160
agtacagata tgagggcaaa atcaccaatt attttcacaa gacactggcc cacgtcccgg 8220
aaattatcga gagggacggc tccattgggg catgggctag cgagggaaat gagtctggga 8280
acaagctgtt caggcgcttc cgaaaaatga atgccaggca gtccaagtac tatgaaatgg 8340
aagatgtttt gaaacatcac tggttgtaca cctccaaata cctgcagaag tttatgaatg 8400
ctcataaagc atttaaaaac tcagggttta ccataaactt gcagagaagc tcaggggaca 8460
cattagacct agagaactct ccagaatctc aagatttgat ggaattttaa gcagggaagt 8520
tgcctaggca ttggttttgc aaatgggttt tctctgggtt gcatggagag cttctcctgg 8580
cacccttaac tcctgggtgt ggggcttcac cacccaagtg gtggtaggtt gatgaggcca 8640
gagatggtga acccatgtcg ggaataggta actggtgagc cgattgcttg agctgtttag 8700
ggagttcaga aaagcaatag gagaaaccag ttatttgaaa gctccatagc tgagaacagg 8760

ggtaactgca ggggaccaga ggtgaacaaa gatgtgtgga gaggattgag tgatgccaaa 8820

gtcaaagcca aggttgtcaa aggacagcca gtgaagccag aaaaggaatt tgacttgtgg 8880

tttccattcc ccctgcccaa gttattctag tttatactga gaggtgtttt tcatatcact 8940

tttgtagaat tttttatttt ataaatttca catattttgg cttattttac aagatttaaa 9000

aaaggttttc aagaattccc gctgtggcac aatgtggcat cttgggagtg ctgggacaca 9060

ggttccatcc ccagcctggc gtggtgggtt aaggatcc 9098

<210> 2

<211> 3831

<212> DNA

<213> Sus. scrofa

<220>

<221> CDS

<222> (113)..(3244)

<400> 2

gggatgggag agcacatttt gccttctctt tggtagcgag taatatcaac cgaagtgcag 60

acatcctaac actttgccca ggcagcctgc tgagcaaggt acctcagcca gc atg gct 118

Met Ala

1

gtc tct ttg cca ccc act ctg gga ctc agt tcc gcc cca gat gaa atc 166

Val Ser Leu Pro Pro Thr Leu Gly Leu Ser Ser Ala Pro Asp Glu Ile

        5                   10                  15

cag cac ccc cac att aaa ttt tca gaa tgg aaa ttt aag cta ttc agg 214
Gln His Pro His Ile Lys Phe Ser Glu Trp Lys Phe Lys Leu Phe Arg
    20                  25                  30

gtg aga tcc ttt gaa aag gca cct gaa aag gct caa acg gaa aag cag 262
Val Arg Ser Phe Glu Lys Ala Pro Glu Lys Ala Gln Thr Glu Lys Gln
35                  40                  45                  50

gat tcc tcc gag ggg aaa ccc tcg ctg gag caa tct cca gca gtc ctg 310
Asp Ser Ser Glu Gly Lys Pro Ser Leu Glu Gln Ser Pro Ala Val Leu
                55                  60                  65

gac aag cct ggt ggt cag aag tca gcc ctg cct caa cca gca ttc aag 358
Asp Lys Pro Gly Gly Gln Lys Ser Ala Leu Pro Gln Pro Ala Phe Lys
            70                  75                  80

ccc cat cca aag ttt tta aag gaa tcc cac gaa gat ggg aaa gca aga 406
Pro His Pro Lys Phe Leu Lys Glu Ser His Glu Asp Gly Lys Ala Arg
        85                  90                  95

gac aaa gcc atc cac caa gcc aac ctg aga cgt ctc tgc cgc atc tgt 454
Asp Lys Ala Ile His Gln Ala Asn Leu Arg Arg Leu Cys Arg Ile Cys
    100                 105                 110

gggaat tct ttc aac acc act ggg cac aag aga agg tat cca gtc cac 502

Gly Asn Ser Phe Asn Thr Thr Gly His Lys Arg Arg Tyr Pro Val His

115                 120                 125                 130

ggg cct gtg gat ggt aaa acc caa gtc ctt tta cgg aag aag gaa aag 550

Gly Pro Val Asp Gly Lys Thr Gln Val Leu Leu Arg Lys Lys Glu Lys

                135                 140                 145

agg gcc acg tcc tgg cca gac ctc att gcc aaa gtt ttc cgg atc gat 598

Arg Ala Thr Ser Trp Pro Asp Leu Ile Ala Lys Val Phe Arg Ile Asp

            150                 155                 160

gtg aag gca gat gtt gac tcg atc cac ccc act gag ttc tgc cat aac 646

Val Lys Ala Asp Val Asp Ser Ile His Pro Thr Glu Phe Cys His Asn

        165                 170                 175

tgc tgg agc ttc atg cac agg aag ttt agc agc acc cca tgt gag gtt 694

Cys Trp Ser Phe Met His Arg Lys Phe Ser Ser Thr Pro Cys Glu Val

    180                 185                 190

tac tcc cca agg aat gca gcc atg gag tgg cac ccc cac acc cta aac 742

Tyr Ser Pro Arg Asn Ala Ala Met Glu Trp His Pro His Thr Leu Asn

195                 200                 205                 210

tgt gac atc tgc cac att gca cgt cgg gga ctc aag agg aag agt cag 790

Cys Asp Ile Cys His Ile Ala Arg Arg Gly Leu Lys Arg Lys Ser Gln

                215                 220                 225

cag cca aac atg cag ctc agc aaa aaa ctc aaa act gtg att gac cga 838
Gln Pro Asn Met Gln Leu Ser Lys Lys Leu Lys Thr Val Ile Asp Arg
            230                 235                 240

gcg aga caa gcc cgt cag cgc aag agg aga gct cag gcc agg atc agc 886
Ala Arg Gln Ala Arg Gln Arg Lys Arg Arg Ala Gln Ala Arg Ile Ser
        245                 250                 255

agc aag gaa ctg atg aag aag atc gcc aac tgc ggt cag ata cat ctt 934
Ser Lys Glu Leu Met Lys Lys Ile Ala Asn Cys Gly Gln Ile His Leu
    260                 265                 270

agc ccc aag ctc ctg gca gtg gac ttc ccg gcg cac ttt gtg aaa tct 982
Ser Pro Lys Leu Leu Ala Val Asp Phe Pro Ala His Phe Val Lys Ser
275                 280                 285                 290

atc tcc tgc cag att tgt gaa cac atc ctg gcc gac ccg gtg gag acc 1030
Ile Ser Cys Gln Ile Cys Glu His Ile Leu Ala Asp Pro Val Glu Thr
                295                 300                 305

agc tgc aag cat gtg ttt tgc agg atc tgc att ctc agg tgc ctc aaa 1078
Ser Cys Lys His Val Phe Cys Arg Ile Cys Ile Leu Arg Cys Leu Lys
            310                 315                 320

gtc atg ggc agc agt tgt ccc tct tgc cac tat ccc tgt ttt cct act 1126
Val Met Gly Ser Ser Cys Pro Ser Cys His Tyr Pro Cys Phe Pro Thr
        325                 330                 335
gac ctg gag agt cca gtg aag tct ttt ctg agc atc ttg aat acc ctg 1174
Asp Leu Glu Ser Pro Val Lys Ser Phe Leu Ser Ile Leu Asn Thr Leu
    340                 345                 350
atg gtg aaa tgc cca gca aag gag tgc aac gag gag atc agc ttg gaa 1222
Met Val Lys Cys Pro Ala Lys Glu Cys Asn Glu Glu Ile Ser Leu Glu
355                 360                 365                 370
aaa tat aat cac cat atc tca agc cac aag gag tcg aag gag aca ttt 1270
Lys Tyr Asn His His Ile Ser Ser His Lys Glu Ser Lys Glu Thr Phe
                375                 380                 385
gtg cat att aat aaa ggg ggc cgg ccc cgc cag cat ctc ctg tcc ctg 1318
Val His Ile Asn Lys Gly Gly Arg Pro Arg Gln His Leu Leu Ser Leu
            390                 395                 400
acg cgg agg gct cag aaa cac cgt ctg agg gag ctc aag ctg caa gtc 1366
Thr Arg Arg Ala Gln Lys His Arg Leu Arg Glu Leu Lys Leu Gln Val
        405                 410                 415
aag gcc ttc gcc gac aaa gaa gaa ggt ggc gat gtg aag tca gtg tgc 1414
Lys Ala Phe Ala Asp Lys Glu Glu Gly Gly Asp Val Lys Ser Val Cys

420                 425                 430

ctg acc ttg ttc ctg cta gtg ctg agg gcg agg aat gag cac aga caa    1462
Leu Thr Leu Phe Leu Leu Val Leu Arg Ala Arg Asn Glu His Arg Gln
435                 440                 445                 450

gct gac gag ctg gag gcc atc atg cga ggc cag ggc tcc ggc ctg cag    1510
Ala Asp Glu Leu Glu Ala Ile Met Arg Gly Gln Gly Ser Gly Leu Gln
                455                 460                 465

cct gct gtt tgc ttg gcc atc cgc gtc aac acc ttc ctc agc tgc agc    1558
Pro Ala Val Cys Leu Ala Ile Arg Val Asn Thr Phe Leu Ser Cys Ser
            470                 475                 480

cag tac cac aag atg tac agg act gtg aag gcc atc acg ggc agg cag    1606
Gln Tyr His Lys Met Tyr Arg Thr Val Lys Ala Ile Thr Gly Arg Gln
        485                 490                 495

att ttc cag cct ttg cat gcc ctt cgg aat gcg gag aag gtc ctt ctg    1654
Ile Phe Gln Pro Leu His Ala Leu Arg Asn Ala Glu Lys Val Leu Leu
    500                 505                 510

ccc ggc tac cac ccc ttc gag tgg cag cca cct ctg aag aat gtg tct    1702
Pro Gly Tyr His Pro Phe Glu Trp Gln Pro Pro Leu Lys Asn Val Ser
515                 520                 525                 530

tcc agc aca gac gtg ggc att att gat ggg ctg tct gga ctc tcc tcc 1750
Ser Ser Thr Asp Val Gly Ile Ile Asp Gly Leu Ser Gly Leu Ser Ser
                535                 540                 545

tct gtg gac gat tac cca gtg gac acc att gcc aag cgc ttc cgc tat 1798
Ser Val Asp Asp Tyr Pro Val Asp Thr Ile Ala Lys Arg Phe Arg Tyr
            550                 555                 560

gac tcg gct ctg gtg tcc gct ctc atg gac atg gaa gaa gac atc ctg 1846
Asp Ser Ala Leu Val Ser Ala Leu Met Asp Met Glu Glu Asp Ile Leu
        565                 570                 575

gag ggt atg aga gcc caa gac ctt gac gac tac ctg aat ggc ccc ttc 1894
Glu Gly Met Arg Ala Gln Asp Leu Asp Asp Tyr Leu Asn Gly Pro Phe
    580                 585                 590

act gtg gtg gtg aag gag tct tgt gat ggg atg gga gac gtg agt gag 1942
Thr Val Val Val Lys Glu Ser Cys Asp Gly Met Gly Asp Val Ser Glu
595                 600                 605                 610

aag cac ggc agt ggg ccg gtc gtg ccg gaa aag gcc gtt cgg ttt tcc 1990
Lys His Gly Ser Gly Pro Val Val Pro Glu Lys Ala Val Arg Phe Ser
                615                 620                 625

ttc aca gtc atg aaa atc acc atc gca cac ggg tca cag aac gtg aag 2038
Phe Thr Val Met Lys Ile Thr Ile Ala His Gly Ser Gln Asn Val Lys

            630                 635                 640

gtg ttt gag gaa gcc aag cct aac tct gaa cta tgc tgc aag ccc ttg 2086
Val Phe Glu Glu Ala Lys Pro Asn Ser Glu Leu Cys Cys Lys Pro Leu
        645                 650                 655

tgc ctc atg ctg gcc gac gaa tcc gac cat gag acc ctg acg gcc atc 2134
Cys Leu Met Leu Ala Asp Glu Ser Asp His Glu Thr Leu Thr Ala Ile
    660                 665                 670

ctg agc cct ctc att gcc gag agg gag gcc atg aag agc agc cag cta 2182
Leu Ser Pro Leu Ile Ala Glu Arg Glu Ala Met Lys Ser Ser Gln Leu
675                 680                 685                 690

atg ctg gag atg gga ggc atc ctc cgg act ttc aag ttc atc ttc agg 2230
Met Leu Glu Met Gly Gly Ile Leu Arg Thr Phe Lys Phe Ile Phe Arg
                695                 700                 705

ggc acc gga tat gat gag aaa ctg gtc cgg gaa gtg gaa ggc ctt gag 2278
Gly Thr Gly Tyr Asp Glu Lys Leu Val Arg Glu Val Glu Gly Leu Glu
            710                 715                 720

gct tct ggc tct gtc tac atc tgt acc ctc tgt gat gcc acc cgc ctg 2326
Ala Ser Gly Ser Val Tyr Ile Cys Thr Leu Cys Asp Ala Thr Arg Leu
        725                 730                 735

gaa gcc tct caa aat ctg gtc ttc cac tcc ata acc aga agc cac gcg 2374
Glu Ala Ser Gln Asn Leu Val Phe His Ser Ile Thr Arg Ser His Ala
    740                 745                 750

gag aat ttg gag cgc tat gag gtc tgg cgt tcc aac cca tac cat gag 2422
Glu Asn Leu Glu Arg Tyr Glu Val Trp Arg Ser Asn Pro Tyr His Glu
755                 760                 765                 770

acg gtg gat gaa ctt cgg gac cgg gtg aaa ggg gtc tcg gcc aaa ccc 2470
Thr Val Asp Glu Leu Arg Asp Arg Val Lys Gly Val Ser Ala Lys Pro
                775                 780                 785

ttc att gag acg gtg cct tcc ata gat gcc ctc cac tgt gac att ggc 2518
Phe Ile Glu Thr Val Pro Ser Ile Asp Ala Leu His Cys Asp Ile Gly
            790                 795                 800

aat gca gcc gag ttt tac aag att ttc cag ctc gag ata ggg gag gcg 2566
Asn Ala Ala Glu Phe Tyr Lys Ile Phe Gln Leu Glu Ile Gly Glu Ala
        805                 810                 815

tat aag aac ccc cat gcc tcc aag gag gaa agg aag aga tgg cag gcg 2614
Tyr Lys Asn Pro His Ala Ser Lys Glu Glu Arg Lys Arg Trp Gln Ala
    820                 825                 830

acc ttg gac aag cac ctc cgc aag aag atg aat ctg aag ccc atc atg 2662
Thr Leu Asp Lys His Leu Arg Lys Lys Met Asn Leu Lys Pro Ile Met

835                 840                 845                 850

aga atg aat ggc aac ttt gcc agg aag ctc atg acc aaa gag act gtg     2710
Arg Met Asn Gly Asn Phe Ala Arg Lys Leu Met Thr Lys Glu Thr Val
                855                 860                 865

gaa gca gtc tgt gag tta att ccc tcc gag gag agg cat gaa gct ctg     2758
Glu Ala Val Cys Glu Leu Ile Pro Ser Glu Glu Arg His Glu Ala Leu
            870                 875                 880

agg gaa ctg atg gac ctt tac ctg aag atg aag ccc gtc tgg cga tca     2806
Arg Glu Leu Met Asp Leu Tyr Leu Lys Met Lys Pro Val Trp Arg Ser
        885                 890                 895

tca tgc cct gct aaa gag tgc ccg gaa tcc ctc tgc cag tat agt ttc     2854
Ser Cys Pro Ala Lys Glu Cys Pro Glu Ser Leu Cys Gln Tyr Ser Phe
    900                 905                 910

aat tca cag cgt ttt gct gag ctc ctc tcc acc aag ttc aag tac aga     2902
Asn Ser Gln Arg Phe Ala Glu Leu Leu Ser Thr Lys Phe Lys Tyr Arg
915                 920                 925                 930

tat gag ggc aaa atc acc aat tat ttt cac aag aca ctg gcc cac gtc     2950
Tyr Glu Gly Lys Ile Thr Asn Tyr Phe His Lys Thr Leu Ala His Val
                935                 940                 945

ccg gaa att atc gag agg gac ggc tcc att ggg gca tgg gct agc gag    2998
Pro Glu Ile Ile Glu Arg Asp Gly Ser Ile Gly Ala Trp Ala Ser Glu
            950                 955                 960

gga aat gag tct ggg aac aag ctg ttc agg cgc ttc cga aaa atg aat    3046
Gly Asn Glu Ser Gly Asn Lys Leu Phe Arg Arg Phe Arg Lys Met Asn
        965                 970                 975

gcc agg cag tcc aag tac tat gaa atg gaa gat gtt ttg aaa cat cac    3094
Ala Arg Gln Ser Lys Tyr Tyr Glu Met Glu Asp Val Leu Lys His His
    980                 985                 990

tgg ttg tac acc tcc aaa tac ctg cag aag ttt atg aat gct cat aaa    3142
Trp Leu Tyr Thr Ser Lys Tyr Leu Gln Lys Phe Met Asn Ala His Lys
995                 1000                1005                1010

gca ttt aaa aac tca ggg ttt acc ata aac ttg cag aga agc tca ggg    3190
Ala Phe Lys Asn Ser Gly Phe Thr Ile Asn Leu Gln Arg Ser Ser Gly
                1015                1020                1025

gac aca tta gac cta gag aac tct cca gaa tct caa gat ttg atg gaa    3238
Asp Thr Leu Asp Leu Glu Asn Ser Pro Glu Ser Gln Asp Leu Met Glu
            1030                1035                1040

ttt taa gcaggga agttgcctag gcattggttt tgcaaatggg ttttctctgg        3291
Phe

gttgcatgga gagcttctcc tggcaccctt aactcctggg tgtggggctt caccacccaa 3351

gtggtggtag gttgatgagg ccagagatgg tgaacccatg tcgggaatag gtaactggtg 3411

agccgattgc ttgagctgtt tagggagttc agaaaagcaa taggagaaac cagttatttg 3471

aaagctccat agctgagaac aggggtaact gcaggggacc agaggtgaac aaagatgtgt 3531

ggagaggatt gagtgatgcc aaagtcaaag ccaaggttgt caaaggacag ccagtgaagc 3591

cagaaaggaa tttgacttgt ggtttccatt ccccctgccc aagttattct agtttatact 3651

gagaggtgtt tttcatatca cttttgtaga attttttatt ttataaattt cacatatttt 3711

ggcttatttt acaagattta aaaaaggttt tcaagaattc ccgctgtggc acaatgtggc 3771

atcttgggag tgctgggaca caggttccat ccccagcctg gcgtggtggg ttaaggatcc 3831

<210> 3

<211> 1043

<212> PRT

<213> Sus. scrofa

<400> 3

Met Ala Val Ser Leu Pro Pro Thr Leu Gly Leu Ser Ser Ala Pro Asp
1               5                   10                  15

Glu Ile Gln His Pro His Ile Lys Phe Ser Glu Trp Lys Phe Lys Leu
            20                  25                  30

Phe Arg Val Arg Ser Phe Glu Lys Ala Pro Glu Lys Ala Gln Thr Glu
        35                  40                  45

Lys Gln Asp Ser Ser Glu Gly Lys Pro Ser Leu Glu Gln Ser Pro Ala
    50                  55                  60

Val Leu Asp Lys Pro Gly Gly Gln Lys Ser Ala Leu Pro Gln Pro Ala
65                  70                  75                  80

Phe Lys Pro His Pro Lys Phe Leu Lys Glu Ser His Glu Asp Gly Lys
                85                  90                  95

Ala Arg Asp Lys Ala Ile His Gln Ala Asn Leu Arg Arg Leu Cys Arg
            100                 105                 110

Ile Cys Gly Asn Ser Phe Asn Thr Thr Gly His Lys Arg Arg Tyr Pro
        115                 120                 125

Val His Gly Pro Val Asp Gly Lys Thr Gln Val Leu Leu Arg Lys Lys
    130                 135                 140

Glu Lys Arg Ala Thr Ser Trp Pro Asp Leu Ile Ala Lys Val Phe Arg
145                 150                 155                 160

Ile Asp Val Lys Ala Asp Val Asp Ser Ile His Pro Thr Glu Phe Cys
                165                 170                 175

His Asn Cys Trp Ser Phe Met His Arg Lys Phe Ser Ser Thr Pro Cys
            180                 185                 190

Glu Val Tyr Ser Pro Arg Asn Ala Ala Met Glu Trp His Pro His Thr
        195                 200                 205

Leu Asn Cys Asp Ile Cys His Ile Ala Arg Arg Gly Leu Lys Arg Lys
    210                 215                 220

Ser Gln Gln Pro Asn Met Gln Leu Ser Lys Lys Leu Lys Thr Val Ile
225                 230                 235                 240

Asp Arg Ala Arg Gln Ala Arg Gln Arg Lys Arg Arg Ala Gln Ala Arg
                245                 250                 255

Ile Ser Ser Lys Glu Leu Met Lys Lys Ile Ala Asn Cys Gly Gln Ile
            260                 265                 270

His Leu Ser Pro Lys Leu Leu Ala Val Asp Phe Pro Ala His Phe Val

        275                 280                 285

Lys Ser Ile Ser Cys Gln Ile Cys Glu His Ile Leu Ala Asp Pro Val

    290                 295                 300

Glu Thr Ser Cys Lys His Val Phe Cys Arg Ile Cys Ile Leu Arg Cys

305                 310                 315                 320

Leu Lys Val Met Gly Ser Ser Cys Pro Ser Cys His Tyr Pro Cys Phe

                325                 330                 335

Pro Thr Asp Leu Glu Ser Pro Val Lys Ser Phe Leu Ser Ile Leu Asn

            340                 345                 350

Thr Leu Met Val Lys Cys Pro Ala Lys Glu Cys Asn Glu Glu Ile Ser

        355                 360                 365

Leu Glu Lys Tyr Asn His His Ile Ser Ser His Lys Glu Ser Lys Glu

    370                 375                 380

Thr Phe Val His Ile Asn Lys Gly Gly Arg Pro Arg Gln His Leu Leu

385                 390                 395                 400

Ser Leu Thr Arg Arg Ala Gln Lys His Arg Leu Arg Glu Leu Lys Leu

                405                 410                 415

Gln Val Lys Ala Phe Ala Asp Lys Glu Glu Gly Gly Asp Val Lys Ser
            420                 425                 430
Val Cys Leu Thr Leu Phe Leu Leu Val Leu Arg Ala Arg Asn Glu His
        435                 440                 445
Arg Gln Ala Asp Glu Leu Glu Ala Ile Met Arg Gly Gln Gly Ser Gly
    450                 455                 460
Leu Gln Pro Ala Val Cys Leu Ala Ile Arg Val Asn Thr Phe Leu Ser
465                 470                 475                 480
Cys Ser Gln Tyr His Lys Met Tyr Arg Thr Val Lys Ala Ile Thr Gly
                485                 490                 495
Arg Gln Ile Phe Gln Pro Leu His Ala Leu Arg Asn Ala Glu Lys Val
            500                 505                 510
Leu Leu Pro Gly Tyr His Pro Phe Glu Trp Gln Pro Pro Leu Lys Asn
        515                 520                 525
Val Ser Ser Ser Thr Asp Val Gly Ile Ile Asp Gly Leu Ser Gly Leu
    530                 535                 540
Ser Ser Ser Val Asp Asp Tyr Pro Val Asp Thr Ile Ala Lys Arg Phe
545                 550                 555                 560

Arg Tyr Asp Ser Ala Leu Val Ser Ala Leu Met Asp Met Glu Glu Asp
                565                 570                 575

Ile Leu Glu Gly Met Arg Ala Gln Asp Leu Asp Asp Tyr Leu Asn Gly
            580                 585                 590

Pro Phe Thr Val Val Val Lys Glu Ser Cys Asp Gly Met Gly Asp Val
        595                 600                 605

Ser Glu Lys His Gly Ser Gly Pro Val Val Pro Glu Lys Ala Val Arg
    610                 615                 620

Phe Ser Phe Thr Val Met Lys Ile Thr Ile Ala His Gly Ser Gln Asn
625                 630                 635                 640

Val Lys Val Phe Glu Glu Ala Lys Pro Asn Ser Glu Leu Cys Cys Lys
                645                 650                 655

Pro Leu Cys Leu Met Leu Ala Asp Glu Ser Asp His Glu Thr Leu Thr
            660                 665                 670

Ala Ile Leu Ser Pro Leu Ile Ala Glu Arg Glu Ala Met Lys Ser Ser
        675                 680                 685

Gln Leu Met Leu Glu Met Gly Gly Ile Leu Arg Thr Phe Lys Phe Ile

    690                 695                 700

Phe Arg Gly Thr Gly Tyr Asp Glu Lys Leu Val Arg Glu Val Glu Gly
705                 710                 715                 720

Leu Glu Ala Ser Gly Ser Val Tyr Ile Cys Thr Leu Cys Asp Ala Thr
                725                 730                 735

Arg Leu Glu Ala Ser Gln Asn Leu Val Phe His Ser Ile Thr Arg Ser
            740                 745                 750

His Ala Glu Asn Leu Glu Arg Tyr Glu Val Trp Arg Ser Asn Pro Tyr
        755                 760                 765

His Glu Thr Val Asp Glu Leu Arg Asp Arg Val Lys Gly Val Ser Ala
    770                 775                 780

Lys Pro Phe Ile Glu Thr Val Pro Ser Ile Asp Ala Leu His Cys Asp
785                 790                 795                 800

Ile Gly Asn Ala Ala Glu Phe Tyr Lys Ile Phe Gln Leu Glu Ile Gly
                805                 810                 815

Glu Ala Tyr Lys Asn Pro His Ala Ser Lys Glu Glu Arg Lys Arg Trp
            820                 825                 830

Gln Ala Thr Leu Asp Lys His Leu Arg Lys Lys Met Asn Leu Lys Pro
        835                 840                 845
Ile Met Arg Met Asn Gly Asn Phe Ala Arg Lys Leu Met Thr Lys Glu
    850                 855                 860
Thr Val Glu Ala Val Cys Glu Leu Ile Pro Ser Glu Glu Arg His Glu
865                 870                 875                 880
Ala Leu Arg Glu Leu Met Asp Leu Tyr Leu Lys Met Lys Pro Val Trp
                885                 890                 895
Arg Ser Ser Cys Pro Ala Lys Glu Cys Pro Glu Ser Leu Cys Gln Tyr
            900                 905                 910
Ser Phe Asn Ser Gln Arg Phe Ala Glu Leu Leu Ser Thr Lys Phe Lys
        915                 920                 925
Tyr Arg Tyr Glu Gly Lys Ile Thr Asn Tyr Phe His Lys Thr Leu Ala
    930                 935                 940
His Val Pro Glu Ile Ile Glu Arg Asp Gly Ser Ile Gly Ala Trp Ala
945                 950                 955                 960
Ser Glu Gly Asn Glu Ser Gly Asn Lys Leu Phe Arg Arg Phe Arg Lys
                965                 970                 975

Met Asn Ala Arg Gln Ser Lys Tyr Tyr Glu Met Glu Asp Val Leu Lys
            980                 985                 990
His His Trp Leu Tyr Thr Ser Lys Tyr Leu Gln Lys Phe Met Asn Ala
        995                 1000                1005
His Lys Ala Phe Lys Asn Ser Gly Phe Thr Ile Asn Leu Gln Arg Ser
    1010                1015                1020
Ser Gly Asp Thr Leu Asp Leu Glu Asn Ser Pro Glu Ser Gln Asp Leu
1025                1030                1035                1040
Met Glu Phe

## Description

### Technical Field

The present invention relates to porcine RAG-1 genes and uses thereof.

### Background Art

In vertebrates, the acquired immune system is the major force against foreign pathogens. The acquired immune system is an antigen-specific immune condition acquired by the body's immune system as a result of stimulation by foreign substances (antigens) after birth. The acquired immune system is triggered by antigen recognition by T cell antigen receptors, or immunoglobulins produced by B cells. In the process of T cell and B cell differentiation, the genes in an embryonic cell genome are reshuffled and become activated genes, and T cell antigen receptors and immunoglobulins exert acquired immune activity. Dr. Susumu Tonegawa received the 1987 Nobel Prize in Physiology and Medicine for the discovery of gene recombination in the development of the acquired immune system.

Gene recombination is initiated by the cleavage of the Recombination Signal Sequence in genomic genes by the RAG-1 and RAG-2 protein complex (RAG-1 protein is the type 1 product of the Recombination Activation Gene). It is known that in the absence of the RAG-1 gene, T cell antigen receptors and immunoglobulins cannot be produced, resulting in severe combined immunodeficiency diseases, without acquired immunity. In fact, it is known that RAG-1 gene-deficient mice, obtained by knockout methods, cannot produce T cell antigen receptors and immunoglobulins (Mombaerts, P. *et al*., Cell, (1992) 68 (5), 869-77).

Such mice, which do not possess acquired immunity, can conceivably be used for xenotransplants, retrogenerative medicine, and such. However, there are problems in the application of these mice to such fields, since mouse organs are small compared with human organs, and mice are physiologically very different from humans (for example, the life expectancy of a mouse is about two years, and mice cells age more rapidly than human cells).

### Disclosure of the Invention

The present inventors considered that swine, whose body fluid composition and physiological functions are similar to those of humans, and whose generation time is shorter than that of primates, were suitably sized animal for application in xenotransplants, retrogenerative medicine, and such. At this time there had been no reports of RAG-1 gene knockout swine. Identifying the porcine RAG-1 gene was prerequisite to constructing a knockout swine, however, there were also no reports of the isolation of a DNA coding for the porcine RAG-1 protein.

The present invention is based on the considerations described above. The first objective of the present invention is to provide DNA coding for the porcine RAG-1 protein. The present invention also aims to provide a vector expressing this DNA, transformants in which the vector has been introduced, porcine RAG-1 protein and methods for its production, and antibodies which bind to this protein.

Furthermore, an objective of the present invention is to construct swine in which the function of the porcine RAG-1 gene is suppressed. Another objective of the present invention is to provide vectors and cells for use in constructing these swine.

The present inventors obtained BAC clones comprising the porcine RAG-1 gene by screening a porcine genomic DNA library. Regions comprising the RAG-1 gene were subcloned from the BAC clones, and three clones were obtained. Genetic maps of these clones were constructed by determining their nucleotide sequences using sequencing, and by mapping using restriction enzyme digestion. Based on this information, targeting vectors for use in knocking-out the RAG-1 gene were constructed.

Specifically, the region from the *Kpn*I recognition site just before the 5' initiation codon of the RAG-1 gene, to the *Hin*cII recognition site approximately 1.4 Kb downstream of this *Kpn*I site, was replaced with a puromycin resistance gene. The puromycin resistance gene is used for positive selection during gene transfer. This substitution prevents the RAG-1 gene from being expressed. The region for homologous recombination was the region from the *Xho*I recognition site approximately 9.0 Kb upstream of the initiation codon, to the *Xho*I site approximately 2.5 Kb downstream of the initiation codon. The DT-A gene was also inserted for negative selection during gene transfer. Swine without an acquired immune system can be constructed by suppressing the function of the endogenous RAG-1 gene using the constructed targeting vectors.

Therefore, the present invention relates to DNA coding for swine-derived RAG-1 protein and uses thereof, especially use for the construction of swine without an acquired immune system. More specifically, it provides:
(1) a porcine-derived DNA of any one of the following (a) to (c):
   (a) a DNA comprising the nucleotide sequence of SEQ ID NO: 1 or 2;
   (b) a DNA encoding a protein comprising the amino acid sequence of SEQ ID NO: 3;
   (c) a DNA encoding a protein comprising an amino acid sequence in which one or more amino acids in the amino acid sequence of SEQ ID NO: 3 have been added, deleted, substituted, and/or inserted, and also comprising a DNA recombination inducing activity;
(2) a vector comprising the DNA of (1);
(3) a transformed cell containing the DNA of (1), or the vector of (2);
(4) a protein encoded by the DNA of (1);
(5) a method for producing the protein of (4), comprising a step of culturing the transformed cell of (3), and recovering an expressed protein from the media of the culture;
(6) an antibody which binds to the protein of (4);
(7) a vector comprising a DNA sequence which suppresses the function of endogenous porcine RAG-1 gene by homologous recombination;
(8) a porcine cell into which the vector of (7) is introduced;
(9) a method for producing porcine in which endogenous RAG-1 gene function is suppressed, comprising the following steps (a) and (b):
   (a) implanting a nucleus of the cell of (8) into an enucleated egg;
   (b) implanting the enucleated egg obtained in (a) into a swine;
(10) a method for producing a swine with suppressed endogenous RAG-1 gene function, comprising a step of implanting the cell of (8) into the swine.

The present invention provides DNAs encoding porcine RAG-1 protein. In the present invention, "RAG-1 protein" indicates a type 1 product of the RAG protein complex necessary for initiation of gene recombination. In the present invention, RAG-1 protein activity can be measured as the activity of inducing DNA recombination. The activity of inducing DNA recombination can be measured using well known methods (Agawal. A., Eastmanq. M. and Schatz D. G. (1998) Nature, 394. 744-751). The RAG-1 gene sequence and RAG-1 cDNA sequence, which were revealed by the present invention, are indicated as SEQ ID NO: 1 and SEQ ID NO: 2, respectively, and the amino acid sequence of the RAG-1 protein encoded by these DNAs is indicated in SEQ ID NO: 3.

The present invention also includes DNAs coding for variants of the porcine RAG-1 protein. Methods well known to those skilled in the art are used to prepare these proteins, and include known methods of inducing mutations in proteins. For example, one skilled in the art can prepare proteins functionally equivalent to porcine RAG-1 protein (the protein comprising the amino acid sequence described by SEQ ID NO: 3) by inducing appropriate mutations in to its amino acid sequence using site-directed mutagenesis (Hashimoto-Gotoh, T. *et al*., Gene (1995) 152, 271-275; Zoller, MJ, and Smith, M., Methods Enzymol. (1983) 100, 468-500; Kramer, W. *et al*., Nucleic Acids Res. (1984) 12, 9441-9456; Kramer W, and Fritz HJ., Methods. Enzymol. (1987) 154, 350-367; Kunkel, TA, Proc. Natl. Acad. Sci. USA. (1985) 82, 488-492; Kunkel, Methods Enzymol. (1988) 85, 2763-2766). Amino acid mutations can also occur in nature. The proteins of the present invention also include proteins comprising the amino acid sequence of porcine RAG-1 protein in which one or more amino acids are mutated, provided that the resulting mutated proteins are functionally equivalent to porcine RAG-1 protein. The number of amino acids to be mutated in such mutants is generally 50 amino acids or less, preferably 30 amino acids or less, and more preferably ten amino acids or less (for example, five amino acids or less).

A mutated amino acid residue is preferably mutated to a different amino acid that conserves the properties of the amino acid side-chain. Examples of the properties of amino acid side chains include hydrophobic amino acids (A, I, L, M, F, P, W, Y, V), hydrophilic amino acids (R, D, N, C, E, Q, G, H, K, S, T) ; and amino acids comprising an aliphatic side-chain (G, A, V, L, I, P), a hydroxyl group-containing side-chain (S, T, Y), a sulfur atom containing side-chain (C, M), a carboxylic acid- and amide-containing side-chain (D, N, E, Q), a base-containing side-chain (R, K, H), or an aromatic-containing side-chain (H, F, Y, W) (The parenthetic letters indicate the one-letter amino acid codes).

Proteins comprising amino acid sequences that are modified from a given amino acid sequence, by the deletion, addition, and/or substitution with another amino acid, of one or more amino acid residues, are already known to retain their biological activity (Mark, D. F. *et al*., Proc. Natl. Acad. Sci. USA (1984) 81, 5662-5666, Zoller, M. J. & Smith, M., Nucleic Acids Research (1982) 10, 6487-6500, Wang, A. *et al*., Science 224, 1431-1433, Dalbadie-McFarland, G. *et al*., Proc. Natl. Acad. Sci. USA (1982) 79, 6409-6413).

Fusion proteins are included as proteins to which more than one amino acid residue has been added to the porcine RAG-1 protein amino acid sequence. Fusion proteins are fusions of porcine RAG-1 protein and other peptides or proteins, and are included in the present invention. Fusion proteins can be made by techniques well known to those skilled in the art, such as by linking the DNA that encodes porcine RAG-1 protein (comprising the amino acid sequence described in SEQ ID NO: 3) with a DNA encoding another peptide or protein so that their frames match, inserting this fused DNA into an expression vector, and expressing it in a host. There are no restrictions as to the peptides or proteins fused to the protein of the present invention.

Examples of known peptides that can be used as the other peptide, which is fused to porcine RAG-1 protein, include FLAG (Hopp, T. P. *et al*., Biotechnology (1988) 6, 1204-1210), 6 x His containing six His (histidine) residues, 10 x His, HA (Influenza agglutinin), human c-myc fragments, VSP-GP fragments, p18HIV fragments, T7-tag, HSV-tag, E-tag, SV40T antigen fragments, lck tag, α-tubulin fragments, B-tag, Protein C fragments, and the like. Examples of proteins that may be fused to porcine RAG-1 protein include GST (glutathione-S-transferase), HA (Influenza agglutinin), immunoglobulin constant regions, β-galactosidase, MBP (maltose-binding protein), and such. Fusion proteins can be prepared by fusing commercially available DNAs, which encode the above fusion peptides or proteins, with a DNA encoding the protein of the present invention, and expressing the fused DNA thus prepared.

The present invention also provides vectors comprising the above-mentioned DNAs, transformants in which the vectors have been introduced, proteins coded by the above-mentioned DNAs and production method thereof, and antibodies that bind to these proteins.

Vectors generally used by those skilled in the art can be applied to the present invention. For example, if *E. coli* is used as a host for transformation, it is preferable to use, for example, vectors comprising "ori", which is required for replication in *E. coli*, and genes necessary for the selection of transformed *E. coli* (for example, genes resistant to drugs (such as Ampicillin, Tetracycline, Kanamycin, and Chloramphenicol)). These vectors include, for example, M13 type vector, pUC type vector, pBR322, pBluescript, pCR-Script, pGEM-T, pDIRECT, and pT7.

An expression vector is especially useful when a vector is used to produce RAG-1 protein. For example, if aiming for expression in *E. coli*, expression vectors include vectors comprising a lacZ promoter (Ward *et al*., Nature (1989) 341, 544-546; FASEB J (1992) 6, 2422-2427), araB promoter (Better *et al*., Science (1988) 240, 1041-1043), T7 promoter or such. In addition to the above vectors, examples of these kinds of vectors include pGEX-5X-1 (Pharmacia), "QIAexpress system" (Qiagen), pEGFP, and pET (in this case, the host is preferably T7 RNA polymerase-expressing BL21). Additionally, the expression vectors may also contain signal sequences for polypeptide secretion. The pelB signal sequence or the like can be used as the signal sequence for protein secretion when, for example, the protein is to be produced in the periplasm of *E. coli* (Lei, S. P. *et al* J. Bacteriol. (1987) 169, 4379).

Other vectors for producing RAG-1 protein include, for example, expression vectors derived from mammals (for example, pcDNA3 (Invitrogen) and pEGF-BOS (Nucleic Acids. Res. (1990) 18 (17), p5322), pEF, pCDM8), expression vectors derived from insect cells (for example, "Bac-to-BAC baculovirus expression system" (GIBCO BRL), pBacPAK8), expression vectors derived from plants (for example pMH1, pMH2), expression vectors derived from animal viruses (for example, pHSV, pMV, pAdexLcw), expression vectors derived from retroviruses (for example, pZIpneo), expression vector derived from yeast (for example, "Pichia Expression Kit" (Invitrogen), pNV11, SP-Q01), and expression vectors derived from *Bacillus subtilis* (for example, pPL608, pKTH50).

When aiming to express a vector in animal cells, such as CHO, COS, or NIH3T3 cells, the vector should comprise a promoter necessary for expression in such cells, for example, SV40 promoter (Mulligan *et al*., Nature (1979) 277, 108), MMLV-LTR promoter, the EF1αpromoter (Mizushima *et al*., Nucleic Acids Res. (1990) 18, 5322), CMV promoter, or the like, and preferably comprise a marker gene for selecting transformed cells (for example, genes that are resistant to drugs (e.g., neomycin, G418 and such)). Examples of known vectors with these characteristics include, for example, pMAM, pDR2, pBK-RSV, pBK-CMV, pOPRSV, and pOP13.

A method when aiming to express a gene stably and to amplify the gene copy number in cells is, for example, the introduction of a vector comprising a complementary DHFR gene (for example pCHO I) into CHO cells with a defective pathway for nucleic acid synthesis, and then amplifying using methotrexate (MTX). Furthermore, when aiming for transient expression of a gene, a method can be used wherein a vector comprising a SV40 replication origin (pcD, etc.) is transfected into COS cells comprising the SV40 T antigen-expressing gene in their chromosomes.

Moreover, the origin of replication can be derived from a polyomavirus, adenovirus, bovine papilomavirus (BPV), and such. Furthermore, expression vectors can contain aminoglycosid transferase (APH) gene, thymidine kinase (TK) gene, *E. coli* xanthine guanine phosphoribosyl transferase (Ecogpt) gene, dihydrofolic acid reductase (dhfr), and such as selective markers for amplification of gene copy number in a host cell system.

The host cell into which the vector is transfected is not particularly limited. For example, E. coli and various types of eukaryote cells and such can be used. For example, when using eukaryotic cells, animal, plant, or fungi cells can be used as a host. Exemplary animal cells include, for example, mammalian cells such as CHO, COS, 3T3, myeloma, baby hamster kidney (BHK), HeLa, or Vero cells, amphibian cells such as Xenopus oocytes (Valle *et al*. Nature (1981) 291:340-358), or insect cells such as Sf9, Sf21, or Tn5 cells. Particularly suitable CHO cells are DHFR-gene-deficient CHO cells dhfr-CHO (Proc. Natl. Acad. Sci. U.S.A. (1980) 77:4216-4220) or CHO K-1 (Proc. Natl. Acad. Sci. U.S.A. (1968) 60:1275). Of the animal cells, CHO cells are particularly preferable when aiming for abundant expression. *Nicotiana tabacum-*derived cells are examples of the plant cells. Known fungi cells include yeast cells such as *Saccharomyces,* including *Saccharomyces cerevisiae,* or filamentous fungi such as *Aspergillus,* including *Aspergillus niger.* When using prokaryotic cells, examples of the bacterial cells include *E. coli*, for example, JM109, DH5α, HB101, XL1Blue and BL21. In addition, *Bacillus subtilis* is also known.

When using animals as hosts, mammals, plants, and insects can be used. As the mammals, goats, swine, sheep, mice, and bovine can be used (Vicki Glaser, SPECTRUM Biotechnology Applications (1993)). Furthermore, for example, tobacco can be used when using plants. Silkworms can be used as the insects, for example.

In order to construct the transformants of the present invention, vectors are introduced into the above-described hosts. Methods for introducing vectors into host cells such as E. coli include, for example, calcium chloride methods and electroporation methods (Chu, G. *et al*., Nucl. Acid. Res. (1987) 15, 1311-1326). When the host cells are cultured cells, vectors can be introduced by, for example, calcium phosphate methods (Chen, C. and Okayama, H. Mol. Cell. Biol. (1987) 7, 2745-2752), DEAE Dextran methods (Lopata, M. A. *et al*., Nucl. Acid. Res. (1984) 12, 5707-5717., Sussman, D. J. and Milman, G. Mol. Cell. Biol. (1985) 4, 1642-1643), cationic liposome DOTAP methods (Boehringer Mannheim), and lipofectin methods (Derijard, B. Cell (1994) 7, 1025-1037., Lamb, B. T. *et al*., Nature Genetics (1993) 5, 22-30., Rabindran, S. K. *et al*., Science (1993) 259, 230-234).

Furthermore, when introducing DNAs into animals, each DNA can be inserted into an appropriate vector (for example, adenovirus vectors (for example pAdex1cw) and retrovirus vectors (for example pZIPneo), without limitation), and introduced into living bodies using methods such as retrovirus methods, liposome methods, cationic liposome methods, and adenovirus. Moreover, vectors can be introduced into insects by, for example, infecting silkworms with a baculovirus comprising a DNA that encodes a target protein (Susumu, M. *et al*., Nature (1985) 315, 592-594). Moreover, when the DNA is to be introduced into plants, for example, DNAs encoding target proteins can be inserted into plant expression vectors such as pMON 530, and introduced into bacteria such as *Agrobacterium tumefaciens.* The vectors can be introduced by infecting these bacteria into tobacco, for example, *Nicotiana tabacum* (Julian K.-C. Ma *et al*., Eur. J. Immunol. (1994) 24, 131-138).

The RAG-1 proteins of the present invention can be produced, for example, by culturing transformants of the present invention. Cultures can be carried out according to known methods. For example, culture media that can generally be used when culturing animal cells include DMEM, MEM, RPMI1640, or IMDM. These media may be used with or without serum supplements such as fetal calf serum (FCS). The pH of the culture medium is preferably between about 6 and 8. Such cells are typically cultured at about 30°C to 40°C for about 15 to 200 hours, and culture media may be replaced, aerated, or stirred as necessary.

The RAG-1 proteins of the present invention may be isolated from inside or outside (such as from the medium) of host cells, and can be purified as substantially pure homogeneous proteins. General methods for protein isolation and purification can be used without limitation. For instance, column chromatography, filtration, ultrafiltration, salt precipitation, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the protein. Alternatively, protein purification can also be performed by using these columns in multiple combinations.

Examples of chromatography include, for example, affinity chromatography in which antibodies against RAG-1 proteins of the present invention are immobilized, ion-exchange chromatography, hydrophobic chromatography, gel filtration, reverse phase chromatography, adsorption chromatography, and such (Strategies for Protein Purification and Characterization: A Laboratory Course Manual. Ed. Daniel R. Marshak *et al*., Cold Spring Harbor Laboratory Press (1996)). These chromatographies may be performed by liquid chromatography, such as HPLC and FPLC. By use of these purification methods, RAG-1 protein of the present invention can be highly purified.

A protein of the present invention may be optionally modified or partially deleted by treatment with an appropriate protein modification enzyme before or after purification. Useful protein modification enzymes include, but are not limited to, trypsin, chymotrypsin, lysylendopeptidase, protein kinase, glucosidase, and so on.

When RAG-1 proteins of the present invention are expressed in host cells (for example, animal cells and *E. coli*) as fusion proteins with Glutathione S-transferase protein, or as recombinant proteins in which multiple histidine residues are attached, the expressed recombinant proteins can be purified using a glutathione column or a nickel column. After purifying the fusion proteins, regions other than the target protein can be cleaved with thrombin, factor Xa, or such, and removed as necessary.

Furthermore, proteins can also be recovered from systems in which individuals produce a protein. Systems in which proteins are produced by individual bodies include, for example, production systems using animals and plants. Proteins can be recovered after introducing target DNAs into animals or plants, and producing the proteins in the animal or plant bodies.

For instance, a desired DNA may be prepared as a fusion gene, by fusing it with a gene that encodes a protein specifically produced in milk, such as goat β casein gene. DNA fragments comprising the fusion gene are injected into goat embryos, which are then impregnated into female goats. Target proteins can be recovered from milk produced by the transgenic goats (i.e., born from goats that received the modified embryos) or from their offspring. To increase the amount of protein-comprising milk produced by the transgenic goats, appropriate hormones may also be applied to these goats (Ebert K. M. *et al*. Bio/Technology (1994) 12:699-702).

When using insects, target proteins can also be recovered from, for example, the body fluids of silkworms infected with a Baculovirus comprising a DNA encoding a target protein, (Susumu, M *et al*., Nature (1985) 315, 592-594).

Furthermore, when using plants, desired proteins can be recovered from, for example, tobacco (tobacco leaves) regenerated using methods well known to those skilled in the art (Toki *et al*., Plant Physiol (1995) 100, 1503-1507) from *Nicotiana tabacum*-derived cells in which a vector has been introduced by an above-mentioned method (Julian K.-C. Ma *et al*., Eur. J. Immunol. (1994) 24, 131-138).

Antibodies which bind to the RAG-1 proteins of the present invention can be used for purification and detection of RAG-1 protein. Antibodies used for the purification and detection of RAG-1 protein in the present invention can be produced by known methods. There is no particular limit to the form of the antibodies, which include antiserum obtained by immunizing immune animals such as rabbits with antigenic protein, all classes of polyclonal and monoclonal antibodies, as well as human antibodies and humanized antibodies produced by genetic recombination. Furthermore, antibodies can be antibody fragments and modified antibodies as long as they bind to RAG-1 protein.

Antibodies can be obtained by using RAG-1 protein as a sensitizing antigen. RAG-1 proteins for use as sensitizing antigens can be either full-sized proteins or partial peptides of proteins. Moreover, protein-expressing cells or their lysates, or chemically synthesized RAG-1 proteins can be used as sensitizing antigens. Short peptides can be used as antigens by properly binding to carrier proteins such as keyhole limpet hemocyanin, bovine serum albumin, or Ovalbumin.

The mammals immunized by sensitizing antigens are not limited, however, they are preferably selected after consideration of their suitability with the parent cells used for cell fusion in monoclonal antibody production. Animals generally used include rodents such as mice, lagomorphs such as rabbits, or primates such as rhesus monkeys. Methods for immunizing animals with sensitizing antigens are well known to those skilled in the art.

Polyclonal antibodies, for example, may be obtained by collecting blood from antigen-sensitized mammals in which an increase in desired antibodies in the serum has been confirmed, and separating serum from this blood using conventional methods. Serum comprising polyclonal antibodies can be used as the polyclonal antibodies, and fractions comprising polyclonal antibodies may be further isolated from this serum as necessary.

To prepare monoclonal antibodies, for example, an increase in desired antibodies is confirmed in the serum of the above-mentioned antigen-sensitized mammals, and then immune cells are collected and subjected to cell fusion. In this case, spleen cells are specific examples of immune cells preferably used for cell fusion. Other parent cells to be fused with the above immune cells preferably include, for example, mammalian myeloma cells, and more preferably myeloma cells that have acquired a property for selecting fused cells using drugs. The above immune cells and myeloma cells can be fused according to known methods, for example, the method of Milstein *et al*. (Galfre, G. and Milstein, C., Methods Enzymol. (1981) 73, 3-46).

Next, using known methods, hybridomas that produce the desired antibodies are cloned from the hybridomas obtained by cell fusion. The cloned hybridomas are implanted into mouse peritoneal cavities, and then ascitic fluid is recovered from the mice.

Obtained antibodies may be purified to homogeneity. Antibody separation and purification can be performed according to separation and purification methods used for general proteins. For example, the antibodies may be separated and isolated by the appropriate selection and combined use of column chromatography such as affinity chromatography, filtration, ultrafiltration, salting-out, dialysis, SDS polyacrylamide gel electrophoresis, isoelectric focusing, and others (Antibodies: A Laboratory Manual. Ed Harlow and David Lane, Cold Spring Harbor Laboratory (1988)), but these methods are not limiting. Protein A and protein G columns can be used as columns for affinity chromatography. Exemplary protein A columns include, for example, Hyper D, POROS, and Sepharose F.F. (Pharmacia).

For example, the antigen-binding activities of the antibodies can be measured by methods such as absorbance measurement, enzyme-linked immunosorbent assays (ELISA), enzyme immunoassays (EIA), radioimmunoassays (RIA), and/or immunofluorescence. In ELISA, the antibodies are immobilized on a plate, RAG-1 protein is added to the plate, and then a sample comprising a desired antibody, for example the culture supernatant of antibody-producing cells or purified antibodies, is added. A secondary antibody, which recognizes the primary antibody and is labeled with an enzyme such as alkaline phosphatase, is then added, and the plate is incubated. Next, after washing, an enzyme substrate such as *p*-nitrophenyl phosphate is added to the plate, and absorbance is measured to evaluate the sample's antigen-binding activity. Fragments of RAG-1 protein can also be used as a protein that is added to the plates.

The present invention also provides vectors (targeting vectors) that comprise DNA sequences which suppress endogenous porcine RAG-1 gene function by homologous recombination. Individual swine in which endogenous RAG-I gene function is suppressed (the RAG-1 gene is knocked out) can be constructed using these vectors. Suppression of RAG-1 gene function by such vectors can be performed by suppressing the expression of the RAG-1 gene, by inducing a mutation in the expression control region of the RAG-1 gene. Furthermore, this suppression can be performed by inducing a mutation in the exon region of the RAG-1 gene to express a mutant RAG-1 protein, whose activity is suppressed compared with the activity of wild type RAG-1 protein. "Suppression" of RAG-1 gene function includes both complete and partial suppression of the RAG-1 gene.

The vectors that comprise a DNA sequence which suppresses porcine endogenous RAG-1 gene function by homologous recombination are not limited, as long as they are able to induce mutations which cause suppression of endogenous porcine RAG-1 gene function. These vectors include, for example, vectors which comprise a mutated expression control region or exon region of the porcine RAG-1 gene, and/or a homologous recombination region. Moreover, vectors additionally comprising a gene for positive selection (selection to choose only cells in which DNA is introduced) or for negative selection (selection to exclude non-homologous recombinants) are also included. Furthermore, vectors which also comprise a sequence coding for an enzyme which enhances recombination in the RAG-1 gene site, for example, Cre for Cre-lox, are also included.

Induction of mutations can be performed, for example, by using a gene for positive selection as described in the Examples, however, there is no limit to the induction of mutations as long as endogenous RAG-1 gene function can be suppressed. There is no specific limitation as to the genes used for positive selection, and examples include puromycin resistance gene and neomycin resistance gene. Genes used for negative selection include thymidine kinase gene and diphtheria toxin gene.

In the present invention, a homologous recombination region indicates a DNA sequence which mediates homologous recombination when a mutated expression control region or exon region of a porcine RAG-1 gene is introduced into genomic DNA. Therefore, there is no limit to the homologous recombination region in the present invention, as long as the DNA sequence can mediate homologous recombination. Those skilled in the art can ordinarily carry out selection of DNA sequences.

Moreover, the present invention provides porcine cells in which targeting vectors of the present invention are introduced. The porcine cells include somatic cells, fertilized eggs, and such, but are not limited thereto.
In the present invention, methods known to those skilled in the art can be used to introduce targeting vectors into the above-mentioned cells. Specifically, electroporation methods can be performed. For example, 1x 10⁷ cells and 50 nM targeting vector are suspended in 500 µl HEPES buffer and transferred into a 4.0 mm gapped electroporation cuvette. A gene is introduced into the cell by applying one pulse using an electroporator at a setting of 250V, 960 µF, and infinite resistance (values can vary depending on cell origin).

Moreover, the present invention provides methods to produce RAG-1 gene knockout swine (endogenous RAG-1 gene function is suppressed). These methods are not particularly limited, and for example include methods where an enucleated egg is implanted with a nucleus from a somatic cell in which endogenous RAG-1 gene function is suppressed, and the obtained enucleated egg is then implanted into swine, which can give birth to individual swine. Other methods include methods of directly injecting a targeting vector of the present invention into fertilized eggs, and then implanting the fertilized eggs into swine, which can give birth to individual swine.

### Brief Description of the Drawings

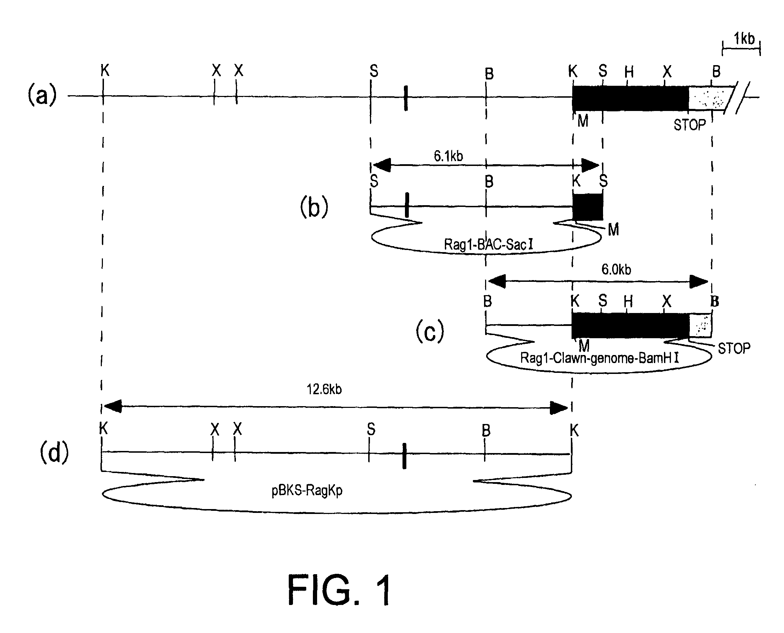

Fig. 1 is a schematic illustration of a vector in which the RAG-1 gene has been subcloned. K, X, S, H, B, and N indicate *Kpn*I, *Xho*I, *Sac*I, *Hin*cII, *Bam*HI, and *Not*I, respectively. (a) indicates genomic DNA, (b) indicates RAG-1-BAC-*Sac*I, (c) indicates Rag1-Clawn-genome-*Bam*HI, (d) indicates pBKS-RagKp.

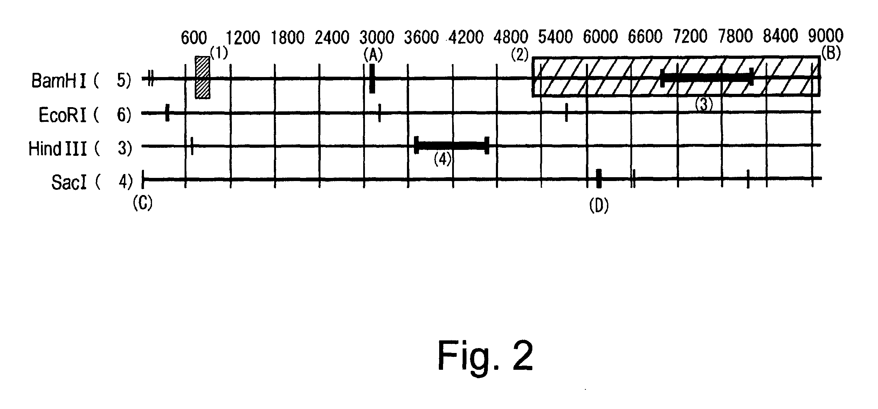

Fig. 2 is a schematic illustration of a genetic map of the RAG-1 gene region. The diagonal box (1) indicates the first exon, diagonal box (2) indicates the second exon, and the region between (1) and (2) indicates an intron. The thick line (3) indicates the region used for the newly devised probe (e.g. RAG-1 Koitabashi probe) for cloning. The thick line (4) indicates the region used as a probe for subcloning from BAC (intron 5' upstream probe). The region between (A) and (B) indicates the Clawn-genome-*Bam*HI subcloning site, and the region between (C) and (D) indicates the BAC-*Sac*I subcloning site.

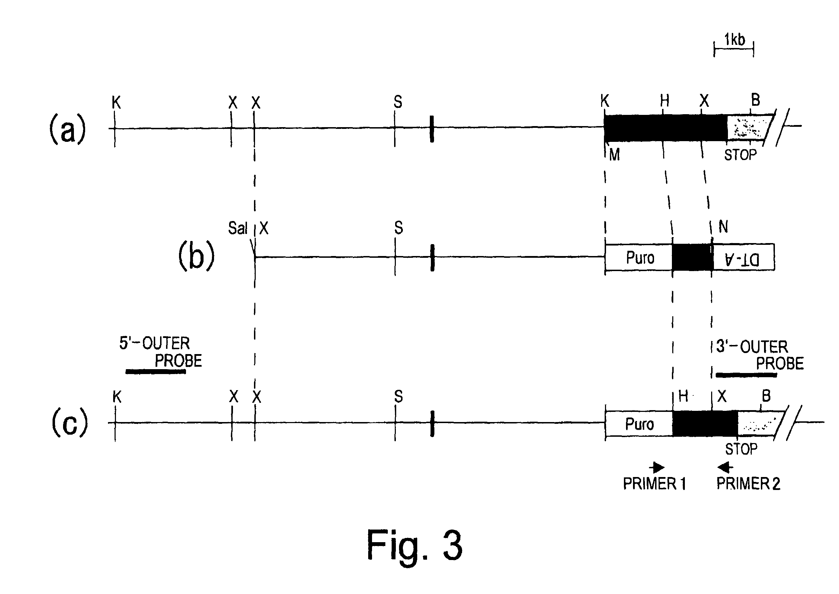

Fig. 3 is a schematic illustration of the targeting vector and a genetic map of the RAG-1 gene region before and after RAG-1 targeting. K, X, S, H, B, Sal, and N indicate *Kpn, Xho*I, *Sac*I, *Hinc*II, *Bam*HI, *Sal*I, and *Not*I, respectively. (a) indicates genomic DNA, (b) indicates a targeting vector, (c) indicates a target genome.

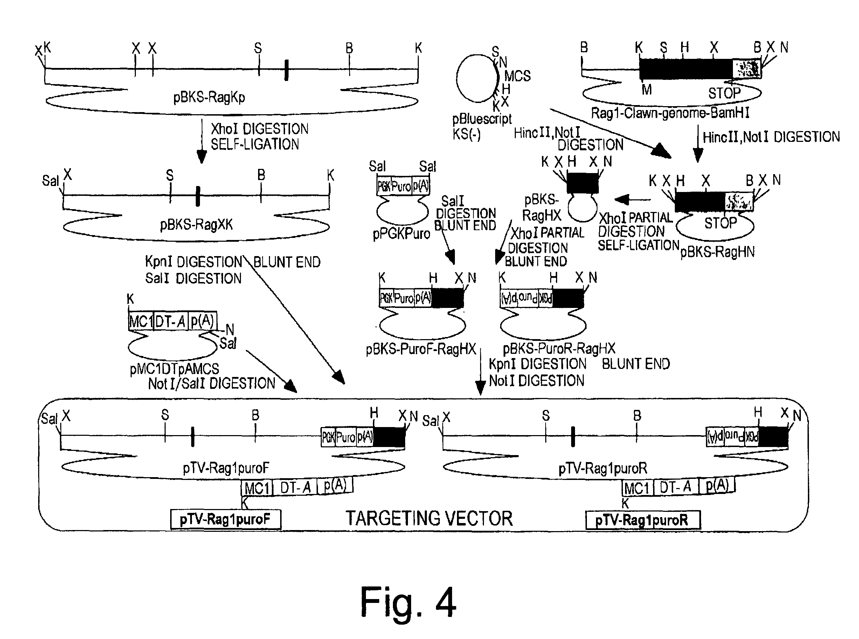

Fig. 4 is a schematic illustration of the process of constructing the RAG-1 gene targeting vectors. K, X, S, H, B, Sal, and N indicate *Kpn*I, *Xho*I, *Sac*I, *Hinc*II, *Bam*HI, *Sal*I, and *Not*I respectively.

### Best Mode for Carrying Out the Invention

The present invention is specifically illustrated below with reference to Examples, but it is not to be construed as being limited thereto.

### [Example 1] Construction of a targeting vector for porcine RAG-1 gene knockout

First, the isolation of porcine RAG-1 cDNA was attempted. At this time, known cDNA could not be obtained. Furthermore, after detailed investigation of information on known mouse and human nucleotide sequences, it was determined that porcine RAG-1 cDNA might not cross-hybridize with probes derived from other species. Therefore, reverse transcription PCR from porcine mRNA was performed. First, four PCR primers known for their use for the human RAG-1 gene were used (Chun *et al*., Cell (1991) 64: 189-200); however, PCR product from swine was not obtained. Therefore, as the result of the trial and error production of a large number of primers, using mice and human nucleotide sequence consensus regions as leads, a 1161 bp cDNA fragment (the region from 7010 bp to 8171 bp in the attached 9.1 Kb nucleotide sequence) with 88% homology to the human RAG-1 cDNA nucleotide sequence was cloned. A porcine thymus cDNA library was screened using this cDNA fragment as a probe, and a cDNA clone covering about 80% of the full-length was obtained. The rest of the cDNA nucleotide sequence was obtained using 5' and 3' RACE methods. Thus, cloning porcine RAG-1 cDNA in sections was extremely difficult.

Next, PCR primers were constructed based on the information obtained from this cDNA. A porcine genomic DNA library was screened using the constructed PCR primers, and BAC clone 578B12, which comprises the porcine RAG-1 gene, was obtained. The region of the BAC clone that comprised the RAG-1 gene was subcloned, and three clones (approximately 6.1 Kb Rag1-BAC-*Sac*I, approximately 6.0 Kb Rag1-Clawn-genome-*Bam*HI, and approximately 12.6 Kb pBKS-RagKp) were obtained (Fig. 1). The nucleotide sequences of these clones were determined by sequencing (approximately 9.1 Kb) (SEQ ID NO: 1), and were mapped by restriction enzyme digests in order to construct an approximately 16 Kb genetic map (Figs. 1 and 2). The targeting vectors for knockout of the RAG-1 gene were constructed based on this information.

Using this targeting vector, the region from the *Kpn*I recognition site, located immediately 5' of the RAG-1 gene initiation codon, to the *Hin*cII recognition site, located approximately 1.4 Kb downstream from the *Kpn*I site, was replaced with a puromycin resistance gene (Fig. 3). This puromycin resistance gene is utilized for positive selection during gene transfer. The RAG-1 gene cannot be expressed because of this substitution. The region for homologous recombination is defined as the region between the *Xho*I recognition site, located approximately 9.0 Kb upstream of the initiation codon, and the *Xho*I recognition site, located approximately 2.5 Kb downstream of this initiation codon. Furthermore, the DT-A gene is linked for use for negative selection during gene transfer (Fig. 3).

First, pBKS-RagKp, which comprised the 5' side of the homologous recombination region, was digested with *Xho*I and self-ligated to obtain pBKS-RagXK. Furthermore, Rag1-Clawn-genome-*Bam*HI, which comprised the 3' side of the homologous recombination region, was digested with *Hin*cII and *Not*I to obtain an approximately 2.2 Kb fragment. pBKS-RagHN was obtained by ligating this fragment to pBluescriptKS(-), which had been digested with *Hin*cII and *Not*I in the same way. This vector was then partially digested with *Xho*I and self-ligated to obtain pBKS-RagHX, in which the approximately 1.2 Kb comprising the stop codon was removed. The approximately 4.0 Kb fragment obtained by partially digesting pBKS-RagHX with *Xh*oI, was ligated to the approximately 1.7 Kb fragment obtained by digesting pPGK-puro, which comprised the puromycin resistance gene, with *Sal*I. Thus, two different types of vectors with opposite orientation of the puromycin resistance gene were obtained: pBKS-PuroF-RagHX and pBKS-PuroR-RagHX. These two vectors were digested with *Kpn*I and *Not*I, and the approximately 2.7 Kb fragment thus obtained was linked with the approximately 9.0 Kb fragment obtained by digesting the above-mentioned pBKS-RagXK with *Sal*I and *Kpn*I, and an approximately 4.3 Kb fragment obtained by digesting a pMC1DTpAMCS vector comprising the DT-A gene with *Not*I and *Sal*I. The targeting vectors pTV-RaglpuroF and pTV-Rag1puroR were thus obtained (Fig. 4).

### Industrial Applicability

Swine without T cell antigen receptors and immunoglobulins can be obtained using the targeting vectors of the present invention. This results in swine that lack acquired immunity, rendering several important applications possible.

### (1) Miniature swine for xenotransplants

At the present time, due to advances in technology, the demand for organs for organ transplant from brain-dead individuals is more than five times that of supply. Therefore, there is a serious undersupply of organs, such that there is even an organ black market. Modified miniature swine that carry human genes for organ transplant into humans have already been developed by several European and American venture companies, and the transplanted condition has been successfully maintained for several months in primates such as baboons. In these miniature swine for use in xenotransplants, rejection that arises at a relatively early stage, such as hyperacute vascular rejection, does not occur. However, in order to maintain transplants for a long period, cellular rejection, which appears gradually, must be understood. Two aspects of the problem of cellular rejection must be examined: that of how a transplant recipient's acquired immune system reacts with the transplanted organ; and the problem of runaway porcine immune function, where the porcine T cells and B cells attached to the transplanted organ proliferate in the transplant recipient's body, and recognize the recipient as a foreign object. The RAG-1 gene in miniature swine for xenograft use is knocked out, and post-transplant transfer of the porcine acquired immune system to recipients does not occur in swine without T cells and B cells. As a result, the success rate of xenotransplantation is expected to rapidly increase.

### (2) RAG-1 knockout swine as human organ incubators for retrogenerative medicine

Nude mice lacking a thymus do not possess acquired immunity, due to T cell antigen receptor deficiency, and hence they do not reject human cells and organs. Therefore, transplant organs for plastic surgery, such as human noses and auricles, have been cultured in nude mice. However, mice are limited in size, and larger organs for retrogenerative medicine use cannot be produced.

The body fluid composition and physiological function of omnivorous swine is more similar to humans than that of other herbivorous livestock animals. Swine also have a shorter generation time than primates. Thus, swine are the most suitable animals to use as incubator bodies for culturing human organs for retrogenerative medicine. Since RAG-1 knockout swine have lost their acquired immunity, they are similar to nude mice in that they do not reject human organs, and can supply nutrients and culture organs.

### (3) Human acquired immunity developed using swine

It is predicted that human acquired immunity can be established by transferring human bone marrow into RAG-1 knockout swine, since they do not have their own acquired immunity. If porcine/human bone marrow chimeric animals for acquired immunity can be developed, it will no longer be necessary to humanize mouse antibodies or the like using conventional recombinant DNA techniques. It will be possible to obtain swine that directly produce human antibodies. With regards to T cells, Human and swine MHC (major histocompatibility antigen complex) class II molecules, which have an important role in T cell antigen recognition, are very similar, and human helper T cells that function in humans can therefore be produced. For example, AIDS virus infections are mediated by the CD4 molecule of helper T cells, causing the death of helper T cells. As a result, AIDS-infected patients lose their acquired immunity and die from opportunistic infections. However, CD4 molecules that resist the human AIDS virus have been already constructed. Therefore, acquired immunity can be restored to AIDS patients by making their helper T cells resistant to the AIDS virus, amplifying these cells in RAG-1 knockout swine, and then transferring the cells back into the patients. Immunity and immune activity can be recovered in patients with immunodeficiency, or with reduced immunity due to aging, by injection of their own acquired immune system cells, which have been amplified inside the body of RAG-1 knockout porcine.

## Claims

1. A porcine-derived DNA of any one of the following (a) to (c):
(a) a DNA comprising the nucleotide sequence of SEQ ID NO: 1 or 2;
(b) a DNA encoding a protein comprising the amino acid sequence of SEQ ID NO: 3;
(c) a DNA encoding a protein comprising an amino acid sequence in which one or more amino acids in the amino acid sequence of SEQ ID NO: 3 have been added, deleted, substituted, and/or inserted, and also comprising a DNA recombination inducing activity.

2. A vector comprising the DNA of claim 1.

3. A transformed cell containing the DNA of claim 1, or the vector of claim 2.

4. A protein encoded by the DNA of claim 1.

5. A method for producing the protein of claim 4, comprising a step of culturing the transformed cell of claim 3, and recovering an expressed protein from the media of the culture.

6. An antibody which binds to the protein of claim 4.

7. A vector comprising a DNA sequence which suppresses the function of endogenous porcine RAG-1 gene by homologous recombination.

8. A porcine cell into which the vector of claim 7 is introduced.

9. A method for producing porcine in which endogenous RAG-1 gene function is suppressed, comprising the following steps (a) and (b) :
(a) implanting a nucleus of the cell of claim 8 into an enucleated egg;
(b) implanting the enucleated egg obtained in (a) into a swine.

10. A method for producing a swine with suppressed endogenous RAG-1 gene function, comprising a step of implanting the cell of claim 8 into the swine.
